# EUROPEAN PATENT APPLICATION

(11) **EP 4 663 237 A1**
(43) Date of publication of application: **17.12.2025**
(21) Application number: 24752731.0
(22) Date of filing: 30.01.2024
(51) Int. Cl.: A61N 1/375

(54) **LEADLESS PACEMAKER AND ASSEMBLY METHOD THEREFOR**

(30) Priority: 06.02.2023 CN 202320170677 U
(71) Applicant: MicroPort Soaring CRM (Shanghai) Co., Ltd., Shanghai 201203 (CN)
(72) Inventor: WU, Nan, Shanghai 201203 (CN)
(74) Representative: Lavoix
(86) International application number: PCT/CN2024/074568
(87) International publication number: WO 2024/164875

(57) **Abstract**

A leadless pacemaker and a method of assembly thereof, the leadless pacemaker includes a pacemaker body, a first base body, a second base body and a connecting member. The first base body is attached to a side of the second base body away from the pacemaker body by the connecting member along an axial direction of the pacemaker body. A first end of the connecting member is attached to the first base body, and a second end of the connecting member is inserted through the second base body and is brought into abutment with an end face of the second base body away from the first base body along the axial direction of the pacemaker body, thereby restricting the first base body from displacement with respect to the second base body along the axial direction of the pacemaker body. With this arrangement, an overlap of the first and second base bodies is fully utilized to effectively enhance axial tensile strength of the leadless pacemaker and attachment strength of its components, thereby satisfying the strength requirements of implantation, retrieval and long-term implantation of the leadless pacemaker without modifying the size thereof while taking into account ease of assembly.

## Description

### TECHNICAL FIELD

The present invention relates to the field of medical devices and, in particular, to a leadless pacemaker and a method of assembly thereof.

### BACKGROUND

Existing leadless pacemakers typically include, among others, a pacemaker distal assembly, a pacemaker body and a pacemaker proximal assembly, which are sequentially coupled together along an axis of the pacemaker. The pacemaker distal assembly is configured to fix the leadless pacemaker to tissue in the heart, and the pacemaker proximal assembly is configured for connection with a delivery device to enable retrieval and deployment of the leadless pacemaker. Since leadless pacemakers are subject to strong axial pulling forces during implantation and retrieval and continuous hemodynamic stress from the surroundings after being implanted, their components are desired to be attached together strongly enough. However, in consideration of electrical communication, sealing, component sizes, ease of assembly and other factors, attachment between some non-metallic components and metallic components often has to essentially rely on resin-based adhesive bonding, which tends to lead to insufficient axial tensile strength of the resulting leadless pacemaker.

### SUMMARY OF THE INVENTION

The present invention seeks to overcome the problem of inadequate inter-component attachment strength associated with conventional leadless pacemakers, by presenting a leadless pacemaker and a method of assembly thereof.

To this end, the present invention provides a leadless pacemaker comprising a pacemaker body, a first base body, a second base body and a connecting member, wherein the first base body is attached to a side of the second base body away from the pacemaker body by the connecting member along an axial direction of the pacemaker body, wherein a first end of the connecting member is attached to the first base body, and wherein a second end of the connecting member is inserted through the second base body and is brought into abutment with an end face of the second base body away from the first base body along the axial direction of the pacemaker body, thereby restricting the first base body from displacement with respect to the second base body along the axial direction of the pacemaker body.

Optionally, in the leadless pacemaker, the connecting member may comprise a first stop surface away from the pacemaker body, wherein the second base body comprises a first through hole configured for insertion of the connecting member therethrough, wherein an end face of the second base body away from the first base body is a second stop surface, wherein the first stop surface is brought into abutment with the second stop surface, thereby restricting the first base body from displacement with respect to the second base body along the axial direction of the pacemaker body.

Optionally, in the leadless pacemaker, the connecting member may comprise an engagement post and an engagement body, wherein the engagement post extends along the axial direction of the pacemaker body, wherein an end of the engagement post away from the pacemaker body is fixedly attached to the first base body, wherein an end of the engagement post facing toward the pacemaker body is fixedly attached to the engagement body, wherein the engagement body and the first base body are located on opposite sides of the second base body, wherein the engagement body extends perpendicularly to the axial direction of the pacemaker body or extends along a circumferential direction of the pacemaker body, wherein the first stop surface is provided at a surface of the engagement body away from the pacemaker body. With this arrangement, depending on different configurations of the engagement body: comprising an engagement achieved by displacement perpendicular to the axial direction of the pacemaker body, or by circumferential rotation, to assemble the first and second base bodies together by a small number of assembly steps with high assembly efficiency.

Optionally, in the leadless pacemaker, when the engagement body extends perpendicularly to the axial direction of the pacemaker body, the first through hole abuts against a side wall of the engagement post, thereby restricting the engagement post from rotation in the first through hole. With this arrangement, in the case of engagement by displacement perpendicular to the axial direction of the pacemaker body, rotation of the engagement post can be restricted. Moreover, even when the engagement is attained by only one engagement body perpendicularly to the axial direction of the pacemaker body, and the engagement direction is on the axis of the pacemaker body, the engagement post can still be restricted from rotation.

Optionally, in the leadless pacemaker, the shape of the first through hole may be a polygon or an ellipse, wherein at least a portion of a circumferential side wall of the engagement post matches the shape of the first through hole.

Optionally, in the leadless pacemaker, the connecting member may further comprise a securing member, wherein the securing member fills a gap between the first through hole and the engagement post, thereby preventing the engagement post from moving perpendicularly to the axial direction of the pacemaker body or moving along a circumferential direction of the pacemaker body within the first through hole . With this arrangement, whether engagement is achieved by displacement perpendicular to the axial direction of the pacemaker body, or by circumferential rotation, the engagement post can be restricted from moving backwards, reducing or preventing dislodgement of the engaged engagement post, once the engagement has been attained.

Optionally, in the leadless pacemaker, the first base body may comprise a third stop surface and a second through hole configured for insertion of the connecting member therethrough, wherein a direction of the second through hole is parallel or perpendicular to the axial direction of the pacemaker body, wherein the connecting member is brought into abutment with the third and second stop surfaces, thereby restricting the first base body from displacement with respect to the second base body along the axial direction of the pacemaker body. With this arrangement, the connecting member is detachable and allows for easier assembly.

Optionally, in the leadless pacemaker, when a direction of the second through hole is parallel to the axial direction of the pacemaker body, the connecting member corresponds to at least one second through hole, wherein the connecting member are successively inserted through corresponding second through hole and the first through hole and is brought into abutment with the second stop surface, wherein a portion of the connecting member located on a side of the first base body and away from second base body is brought into abutment with the third stop surface . With this arrangement, a middle portion of the connecting member can be brought into abutment with the first base body, while end portions thereof can be both brought into abutment with the second base body. This allows for both firmer attachment and ease of assembly.

Optionally, in the leadless pacemaker, the connecting member may be a bendable wire or an elastic engagement element.

Optionally, in the leadless pacemaker, when the connecting member is a bendable wire, two second through holes may correspond to each bendable wire, and opposite ends of the bendable wire may be inserted successively through the respective second through holes and the first through holes and then are bent to form the first stop surface, which is brought into abutment with the second stop surface.

Optionally, in the leadless pacemaker, when the connecting member is an elastic engagement element, the elastic engagement element comprises a base and two elastic legs, wherein the base is brought into abutment with the third stop surface, wherein one end of each leg away from the pacemaker body is attached to base, and the other end of each leg is free end, wherein the free end of the leg comprises a reverse-lock boss extending away from the other leg, and wherein the first stop surface is provided at a surface of the reverse-lock boss away from the pacemaker body. Compared with implementing the connecting member as a bendable wire, implementing it as such an elastic engagement element attached at one end to the first base body and being free at the other end enables assembly more efficiently without involving bending operation(s).

Optionally, in the leadless pacemaker, the direction of the second through hole is perpendicular to the axial direction of the pacemaker body, the second through hole communicates with the first through hole, wherein the connecting member comprises a plug part and a bendable part attached to the plug part, wherein a free end of the bendable part successively inserted through the second and first through holes, and is bended to form the first stop surface brought into abutment with the second stop surface, and wherein the plug part is brought into abutment with the third stop surface. With this arrangement, the connecting member can insert from one side of the first base body successively through the second and first through holes and then bent. This allows for easier assembly and imparts higher shear resistance to the joint of the first and second base bodies.

Optionally, in the leadless pacemaker, the first base body may comprise a recess depressed toward the first base body, wherein the third stop surface is formed at a bottom surface of the recess, wherein the second through hole is provided at the third stop surface, and wherein a portion of the connecting member is received in the recess. With this arrangement, in the case of the connecting member being detachable, the leadless pacemaker is allowed to be made compact.

Optionally, in the leadless pacemaker, the first and second base bodies mate with each other along the axial direction of the pacemaker body in a form of protrusion and recess, thereby restricting the first base body from rotating with respect to the second base body along a circumferential direction of the pacemaker body and/or from displacing with respect to the second base body perpendicularly to the axial direction of the pacemaker body. With this arrangement, protrusion and recess mating of the first and second base bodies can be achieved at the same time as assembly is achieved by the connecting member along the axial direction of the pacemaker body. This allows for easier assembly and imparts higher shear resistance to the joint of the first and second base bodies.

Optionally, in the leadless pacemaker, one of the first and second base bodies comprises a locating protrusion, with the other comprising a locating hole, and wherein the locating protrusion is inserted into the locating hole. With this arrangement, easier fabrication of the structure can be achieved, and inserting the locating protrusion into the locating hole can impart higher shear resistance to the joint of the first and second base bodies.

Optionally, in the leadless pacemaker, one of the first and second base bodies may comprise a circumferential locating boss, with the other comprising a locating depression, wherein the locating boss is inserted into the locating depression. With this arrangement, during assembly of the first and second base bodies, the locating boss and the locating depression can achieve radially and axially locating along the pacemaker body, facilitating subsequent attachment of the connecting member.

Optionally, the leadless pacemaker may comprise at least two connecting members, wherein the first base body is attached to the second base body through the at least two connecting members. With this arrangement, the two or more connecting members can be used to restrict rotation in the case of engagement by movement perpendicular to axial direction of the pacemaker body, and to facilitating even stressing in the case of engagement by circumferential rotation.

Optionally, the leadless pacemaker may further comprise a pacemaker distal assembly, a pacemaker proximal assembly, wherein two first base bodies and two second base bodies are provided, wherein:
axially opposite ends of the pacemaker body are attached to corresponding second base bodies; and
the pacemaker distal assembly is attached to one of the first base bodies located on a distal end of the pacemaker body, and the pacemaker proximal assembly is attached to the other first base body located on a proximal end of the pacemaker body.

To the above end, the present invention also provides a method of assembly of the leadless pacemaker as defined above, which comprises:
bringing the first base body into abutment with one end of the second base body away from the pacemaker body along the axial direction of the pacemaker body; and
inserting the second end of the connecting member through the second base body and bringing the second end of the connecting member into abutment with the end face of the second base body away from the first base body along the axial direction of the pacemaker body, thereby restricting the first base body from displacement with respect to the second base body along the axial direction of the pacemaker body.

Optionally, in the method, inserting the second end of the connecting member through the second base body and bringing the second end of the connecting member into abutment with the end face of the second base body away from the first base body along the axial direction of the pacemaker body may comprise:
moving the first base body perpendicularly to the axial direction of the pacemaker body or along a circumferential direction of the pacemaker body to bring the first stop surface of the engagement body in the connecting member into abutment with the second stop surface of the second base body, thereby restricting the first base body from displacement with respect to the second base body along the axial direction of the pacemaker body. With this arrangement, engagement can be achieved either by displacement perpendicular to the axial direction of the pacemaker body, or by circumferential rotation, to assemble the first and second base bodies together by a small number of assembly steps with high assembly efficiency.

Optionally, after moving the first base body perpendicularly to the axial direction of the pacemaker body or along a circumferential direction of the pacemaker body to bring the first stop surface of the engagement body in the connecting member into abutment with the second stop surface of the second base body, the method may further comprise:
filling a securing member in a gap between a first through hole of the second base body and an engagement post, thereby preventing the engagement post from moving perpendicularly to the axial direction of the pacemaker body or along a circumferential direction of the pacemaker body within the first through hole. With this arrangement, whether engagement is achieved by displacement perpendicular to the axial direction of the pacemaker body, or by circumferential rotation, the engagement post can be restricted from moving backwards, reducing or preventing dislodgement of the engaged engagement post, once the engagement has been attained.

Optionally, in the method, inserting the second end of the connecting member through the second base body and bringing the second end of the connecting member into abutment with the end face of the second base body away from the first base body along the axial direction of the pacemaker body may comprise:
inserting the connecting member successively through the second through hole in the first base body and the first through hole in the second base body, thereby bringing the connecting member into abutment with both the third stop surface of the first base body and the second stop surface. With this arrangement, the connecting member is detachable and allows for easier assembly.

Optionally, in the method, the connecting member may comprise a bendable wire, opposite ends of which are inserted successively through the second and first through holes and then bent into abutment with the second stop surface of the second base body, thereby restricting the first base body from displacement with respect to the second base body along the axial direction of the pacemaker body.

Optionally, in the method, the connecting member may comprise an elastic engagement element comprising two legs, wherein the two legs are inserted successively through corresponding second and first through holes and then elastically deflect away from each other to bring reverse-lock boss of the legs into engagement with the second base body, thereby restricting the first base body from displacement with respect to the second base body along the axial direction of the pacemaker body. Compared with implementing the connecting member as a bendable wire, implementing it as such an elastic engagement element enables assembly more efficiently without involving bending operation(s) during assembly.

Optionally, the method may further comprise performing attachment enhancement by at least one of:
I: a medical adhesive applied between the first and second base bodies along the axial direction of the pacemaker body;
II: when the first through hole is provided in the second base body, a medical adhesive or welding applied between the connecting member and the first through hole;
III: when the second through hole is provided in the first base body, a medical adhesive applied between the connecting member and the second through hole. With this arrangement, through additional strengthened connection, attachment strength of the first and second base bodies can be further enhanced.

As described above, the present invention provides a leadless pacemaker and a method of assembly thereof. The leadless pacemaker includes a pacemaker body, a first base body, a second base body and a connecting member. The first base body is attached to the second base body on a side thereof away from the pacemaker body through the connecting member along an axial direction of the pacemaker body. A first end of the connecting member is attached to the first base body, and a second end of the connecting member is inserted through the second base body and brought into abutment with an end face of the second base body away from the first base body along the axial direction of the pacemaker body, thereby restricting the first base body from displacement with respect to the second base body along the axial direction of the pacemaker body.

With this arrangement, an overlap of the first and second base bodies is to be utilized, allowing for full utilization of the limited space and effectively enhancing the attachment strength of the components of the leadless pacemaker and axial tensile strength of the leadless pacemaker, thereby satisfying the strength requirements of implantation, retrieval and long-term implantation of the leadless pacemaker without modifying the size thereof while taking into account ease of assembly.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 shows a schematic exploded view of a leadless pacemaker according to an embodiment of the present invention;
Fig. 2 schematically illustrates an electrode base in a first example according to an embodiment of the present invention;
Fig. 3 schematically illustrates a distal shell connector in the first example according to an embodiment of the present invention;
Figs. 4a to 4c schematically illustrate assembly and attachment of the first example of the electrode base and distal shell connector according to an embodiment of the present invention;
Figs. 5a and 5b schematically illustrate an assembly aid and assembly and attachment of an electrode base and distal shell connector assisted by the assembly aid according to an embodiment of the present invention;
Fig. 6a schematically illustrates a first example of a proximal connector and safety metal wire connector according to an embodiment of the present invention;
Fig. 6b is a schematic view of the proximal connector and safety metal wire connector of Fig. 6a, taken from a different angle;
Figs. 7a and 7b schematically illustrate assembly and attachment of the first example of the proximal connector and safety metal wire connector according to an embodiment of the present invention;
Fig. 8a schematically illustrates a second example of the electrode base and distal shell connector according to an embodiment of the present invention;
Fig. 8b is a schematic view of the electrode base and distal shell connector of Fig. 8a, taken from a different angle;
Figs. 9a and 9b schematically illustrate assembly and attachment of the second example of the electrode base and distal shell connector according to an embodiment of the present invention;
Fig. 10a schematically illustrates a third example of the electrode base and distal shell connector according to an embodiment of the present invention;
Fig. 10b is a schematic view of the electrode base and distal shell connector of Fig. 10a, taken from a different angle;
Figs. 11a and 11b schematically illustrate assembly and attachment of the third example of the electrode base and distal shell connector according to an embodiment of the present invention;
Fig. 12a schematically illustrates a second example of the proximal connector and safety metal wire connector according to an embodiment of the present invention;
Fig. 12b is a schematic view of the proximal connector and safety metal wire connector of Fig. 12a, taken from a different angle;
Fig. 13 schematically illustrates a connecting member according to an embodiment of the present invention; and
Figs. 14a to 14d schematically illustrate assembly and attachment of the second example of the proximal connector and safety metal wire connector according to an embodiment of the present invention.

In figures
1- pacemaker distal assembly; 11-head electrode; 12-drug plug; 13-fixation helix; 14-fixation helix base; 15-electrode base; 150-first end face; 151-second through hole; 152-third stop surface; 153-recess; 154-first segment; 155-second segment; 156-fourth stop surface; 2-pacemaker body; 21-distal shell connector; 210-second end face; 211-first through hole; 2110-residual space; 212-second stop surface; 22-distal seal end cap; 23-shell; 24-electrical component; 25-battery; 26-ring electrode; 27-proximal seal end cap; 28-safety metal wire connector; 3-pacemaker proximal assembly; 31-proximal connector; 4-connecting member; 401-first stop surface; 411-engagement post; 412-engagement body; 42-elastic engagement element; 421-base; 422-leg; 423-reverse-lock boss; 43-bendable wire; 44-plug part; 441-main body; 442-coupling part; 443- plug end face; 45-bendable part; 5-assembly aid; 50-cavity; 501-stop step; 502-inner stop wall; 61-locating protrusion; 62-locating hole; 63-locating boss; 64-locating depression.

### DETAILED DESCRIPTION

Objects, advantages and features of the present invention will become more apparent upon reading the following more detailed description of specific embodiments thereof in conjunction with the accompanying drawings. Note that the figures are provided in a very simplified form not necessarily drawn to exact scale for the only purpose of helping explain embodiments of the invention disclosed herein in a more convenient and clearer way. In addition, the illustrated structures are usually part of their real-world counterparts. In particular, as the figures tend to have distinct emphases, they are sometimes drawn to different scales.

As used herein, the singular forms "a", "an" and "the" include plural referents, and the term "or" is generally employed in the sense of "and/or", "a number of" is generally employed in the sense of "at least one" and "at least two" is generally employed in the sense of "two or more". Additionally, the use of the terms "first", "second" and "third" herein is intended for illustration only and is not to be construed as denoting or implying relative importance or as implicitly indicating the numerical number of the referenced items. Accordingly, defining an item with "first", "second" or "third" is an explicit or implicit indication of the presence of one or at least two such items. The terms "one end" and "other end", as well as "proximal end" and "distal end", may be used herein to generally refer to corresponding end portions including corresponding endpoints, rather than only to the endpoints. As used herein, the terms "proximal end" and "distal end" may be used with respect to a leadless pacemaker configured for fixation at an implantation site at one end and staying away from the implantation site at the other end. In this regard, when used in relation to a component, the term "proximal end" refers to a location thereon closer to the end of the leadless pacemaker that stays away from the implantation site, and the term "distal end" refers to a location on the component, which is closer to the end of the leadless pacemaker that is fixed at the implantation site and therefore farther away from the end that stays away from the implantation site. The terms "proximal end" and "distal end" may also be used to describe a manual operation, or an operation conducted by hand. In this regard, when used in relation to a component, the term "proximal end" refers to a location thereon closer to an operator, such as a surgeon or clinician, and the term "distal end" refers to a location on the component, which is closer to a leadless pacemaker and therefore farther away from the operator. Further, as used herein, the terms "mounting", "coupling", "connecting", "disposing" and any variants thereof should be interpreted in a broad sense. When an element is referred to as being "disposed" on another element, this is generally intended to only mean that there is a connection, coupling, engagement or transmission relationship between the two elements, which may be either direct or indirect with one or more intervening elements, and should not be interpreted as indicating or implying a particular spatial position relationship between them. That is, the element may be located inside, outside, above, under, beside, or at any other location relative to the other element, unless the context clearly dictates otherwise. Those of ordinary skill in the art can understand the specific meanings of the above-mentioned terms herein, depending on their context. Furthermore, the directional terms such as "above", "below", "upper", "lower", "upward", "downward", "left", "right", and the like are used in relation to the illustrative embodiments as they are depicted in the figures, the upward or upper direction being toward the top of the corresponding figure and the downward or lower direction being toward the bottom of the corresponding figure.

The present invention seeks to overcome the problem of inadequate component attachment strength associated with conventional leadless pacemakers, by presenting a leadless pacemaker and a method of assembly thereof, as detailed below with reference to the annexed figures.

### Embodiment 1

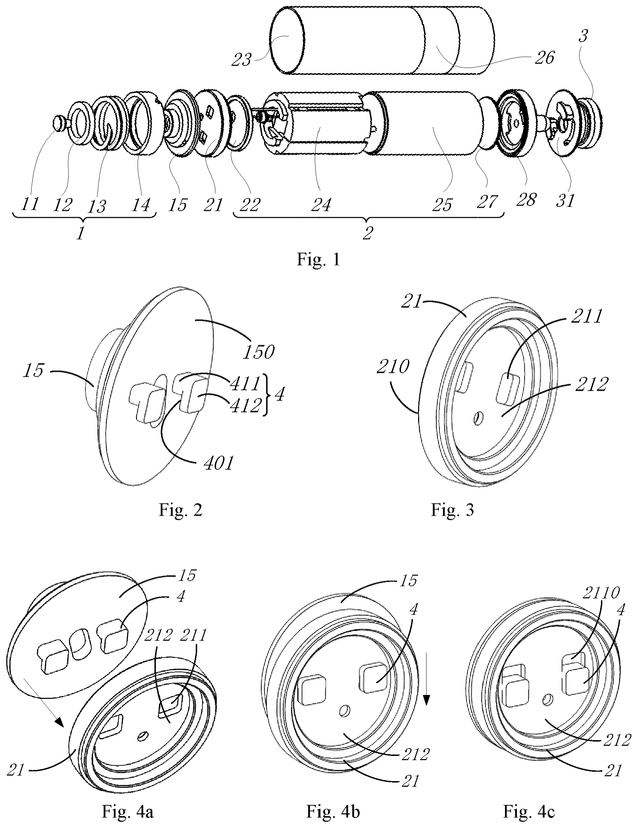

Referring to Figs. 1 to 7b, in a first embodiment of the present invention, there is provided a leadless pacemaker, which includes a pacemaker distal assembly 1, electrode base 15, a distal shell connector 21, a pacemaker body 2, a safety metal wire connector 28, a proximal connector 31 and a pacemaker proximal assembly 3. In one example, the pacemaker distal assembly 1 includes a head electrode 11, a drug plug 12, a fixation helix 13 and a fixation helix base 14. The pacemaker body 2 includes a distal seal end cap 22, a shell 23, an electrical component 24, a battery 25, a ring electrode 26 and a proximal seal end cap 27. The pacemaker proximal assembly 3 includes, among others, a retrieval member for docking with a delivery device. In the pacemaker distal assembly 1, the head electrode 11 is welded and hence electrically connected to the electrical component 24 via a guide wire, and the drug plug 12 is adhesively bonded to the electrode base 15. The fixation helix 13 is attached to the fixation helix base 14 by welding, and the electrode base 15 is coupled to the fixation helix base 14 using metal pins. All these components can be attached with sufficient attachment strength. In the pacemaker body 2, the shell 23 is, for example, a titanium shell. The distal seal end cap 22 is attached to the shell 23 by welding, and both the electrical component 24 and the battery 25 are housed within the shell 23. The ring electrode 26 is disposed over an outer circumferential wall of the shell 23, and the proximal seal end cap 27 is attached to the shell 23 by welding. Further, the distal shell connector 21 is attached to the distal seal end cap 22 by welding, and the safety metal wire connector 28 is attached to the proximal seal end cap 27 by welding. All these components can also be attached with sufficient attachment strength.

Additionally, the distal assembly 1 is attached to a distal end of the pacemaker body 2 through the electrode base 15 and the distal shell connector 21, and the proximal assembly 3 is attached to a proximal end of the pacemaker body 2 through the safety metal wire connector 28 and the proximal connector 31.

However, in consideration of a small size of the pacemaker distal assembly 1 and other factors including ease of assembly and electrical communication, the electrode base 15 is typically made of an insulating material, while the distal shell connector 21 is generally made of a metallic material. The two are generally attached to each other by resin-based adhesive bonding. Additionally, in consideration of a small size of the proximal assembly 3 and other factors including ease of assembly and contrast performance, the proximal connector 31 is generally made of a non-metallic material, while the safety metal wire connector 28 generally of a metallic material.

Conventionally, the two are also generally attached together by resin-based adhesive bonding. However, the connection between the aforementioned two pairs is exposed to pulling forces along the axial direction of the pacemaker body, and a connection strength tends not to meet requirement.

In view of this, in the leadless pacemaker of this embodiment, a connecting member 4 is configured to enhance tensile strength of the joint between the electrode base 15 and the distal shell connector 21, and between the safety metal wire connector 28 and the proximal connector 31 along the axis of the pacemaker body 2. In one implementation, the electrode base 15 is axially attached to the distal shell connector 21, with the connecting member 4 provided therebetween, thereby forming one attached pair. In another implementation, the safety metal wire connector 28 is axially attached to the proximal connector 31, with the connecting member 4 provided therebetween, forming one attached pair. With this arrangement, the added connecting member 4 enables those components of the leadless pacemaker to be attached with sufficient strength, without modifying the overall structure or size of the leadless pacemaker.

For ease of description, referring to Fig. 1, in the following examples, each of the electrode base 15, the distal shell connector 21, the safety metal wire connector 28 and the proximal connector 31 is abstractly considered as a base body. Specifically, in one example, the electrode base 15 is regarded as a first base body and the distal shell connector 21 as a second base body. In an alternative example, the proximal connector 31 is regarded as the first base body and the safety metal wire connector 28 as the second base body. It should be noted that in consideration of material strength, the second base body is made essentially of a metallic material and can be directly welded to the pacemaker body 2, while the first base body is located on the side of the second base body away from the pacemaker body 2 and made essentially of a non-metallic material.

Along the axis of the pacemaker body 2, the first base body is attached to the second base body on the side thereof away from the pacemaker body 2 through the connecting member 4. A first end of the connecting member 4 is attached to the first base body, and a second end of the connecting member 4 passes through the second base body and abut against an end face of the second base body away from first base body along the axis of the pacemaker body 2, thereby restricting displacement of the first base body relative to the second base body along the axis of the pacemaker body 2. With this arrangement, the use of the connecting member 4 enables an overlap of the first and second base bodies to be utilized, allowing for full utilization of the limited space and effectively enhancing the attachment strength of the components of the leadless pacemaker, thereby satisfying the strength requirements of implantation, retrieval and long-term implantation of the leadless pacemaker without modifying the size thereof while taking into account ease of assembly.

Referring to Figs. 2 and 3, in an example, in the context of the electrode base 15 being regarded as the first base body and the distal shell connector 21 as the second base body, the electrode base 15 and the distal shell connector 21 are configured to be attached to each other along the axial direction of the pacemaker body 2. Specifically, the electrode base 15 has a first end face 150 facing toward the distal shell connector 21, and the distal shell connector 21 has a second end face 210 facing toward the electrode base 15. The electrode base 15 and distal shell connector 21 are axially attached to each other, and the first end face 150 and the second end face 210 abut against each other. The connecting member 4 has a first stop surface 401 facing away from the pacemaker body 2, and the distal shell connector 21 has a first through hole 211 for insertion of the connecting member 4 therethrough. An end face of the distal shell connector 21 away from the electrode base 15 provides a second stop surface 212. When the first stop surface 401 comes into abutment with the second stop surface 212, displacement of the electrode base 15 relative to the distal shell connector 21 along the axial direction of the pacemaker body 2 will be restricted.

Optionally, the first stop surface 401 may be configured so as to come into abutment with the second stop surface 212 after the connecting member 4 being inserted through the first through hole 211 and moved in a direction perpendicular to the axis of the pacemaker body 2. Specifically, in the example of Figs. 2 and 3, the connecting member 4 includes an engagement post 411 and an engagement body 412. The engagement post 411 extends along the axial direction of the pacemaker body 2. An end of the engagement post 411 away from the pacemaker body 2 is fixedly attached to the electrode base 15, and the other end thereof facing toward the pacemaker body 2 is fixedly attached to the engagement body 412. The engagement body 412 and the electrode base 15 are located on opposite sides of the distal shell connector 21. The engagement body 412 extends perpendicularly to the axial direction of the pacemaker body 2, and the first stop surface 401 is provided at surface of the engagement body 412 facing away from the pacemaker body 2. The engagement post 411 is configured to move with the electrode base 15, thereby causing movement of the first stop surface 401. It will be understood that, in order to allow the engagement post 411 and the engagement body 412 to be inserted through the first through hole 211, maximum cross-sectional contours of the engagement post 411 and the engagement body 412 (the cutting direction of said cross-section is perpendicular to the axial direction of the pacemaker body 2) should be slightly smaller than an inner contour of the first through hole 211.

Optionally, the engagement body 412 may extend perpendicularly to the axial direction of the pacemaker body 2, and a side wall of the engagement post 411 may abut against the first through hole 211, thus restricting the engagement post 411 from rotation in the first through hole 211. For example, the first through hole 211 may have a non-circular shape, such as polygonal, elliptical or rounded rectangle. At least a portion of the circumferential sidewall of the engagement post 411 matches the hole shape of the first through hole 211. This enables the side wall of the engagement post 411 to abut against the first through hole 211, restricting the engagement post 411 from rotation in the first through hole 211. With this arrangement, in an engagement scheme perpendicular to the axial direction of the pacemaker body 2, the engagement post 411 can be restricted from rotation. Moreover, when only one engagement body 412 is used to perform engagement along a direction perpendicular to the axial direction of the pacemaker body 2 and the engagement direction is on the axis of the pacemaker body 2, the engagement post 411 can still be restricted from rotation.

Preferably, a cross-sectional width of the engagement post 411 perpendicularly to the engagement moving direction matches a cross-sectional width of the first through hole 211 in the engagement moving direction (here, the term "engagement moving direction" refers to a direction perpendicular to the axial direction of the pacemaker body 2, as indicated by the arrow of Fig. 4b). In this way, once the engagement post 411 passes through the first through hole 211, its side wall will come into abutment with the first through hole 211, making the electrode base 15 movable only in the engagement moving direction and restricting the engagement post 411 from rotation in the first through hole 211. Thus, more reliable attachment can be obtained.

In the example of Figs. 2 and 3, the engagement post 411, the engagement body 412 and the first through hole 211 all have rounded rectangle cross-sections (the cutting directions of all cross-sections are perpendicular to the axis of the pacemaker body 2), and the engagement post 411 and the engagement body 412 together have an L-shaped cross-section taken along the axis. After the engagement post 411 and the engagement body 412 pass through the first through hole 211, they can be moved together with the electrode base 15 perpendicularly to the axial direction of the pacemaker body 2, bringing the first stop surface 401 into abutment with the second stop surface 212, as shown in Figs. 4a to 4c. After the first stop surface 401 abuts against the second stop surface 212, any pulling force performed between the electrode base 15 and the distal shell connector 21 along the axial direction of the pacemaker body 2 will be exerted on the L-shaped engagement post 411 and the engagement body 412, which provides much higher tensile strength than attachment established solely by resin-based adhesive bonding.

Optionally, the leadless pacemaker may include at least two connecting members 4, which attach the electrode base 15 to the distal shell connector 21. Accordingly, in this case, the distal shell connector 21 may comprise at least two first through holes 211. In an example, as shown in Figs. 2 and 3, the leadless pacemaker includes two engagement posts 411 and two engagement bodies 412, and the distal shell connector 21 accordingly has two first through holes 211. In the configuration where the connecting member 4 achieves engagement by passing through the first through-hole 211 and subsequently moving perpendicular to the axial direction of the pacemaker body 2, it can also restrict the electrode base 15 and the distal shell connector 21 from rotating circumferentially with respect to the pacemaker body 2. Additionally, the electrode base 15 and the distal shell connector 21 can be attached with even higher strength by the use of at least two connecting members 4. It will be understood that any numbers of engagement posts 411, engagement bodies 412 and first through holes 211 may be included, as desired in practical applications.

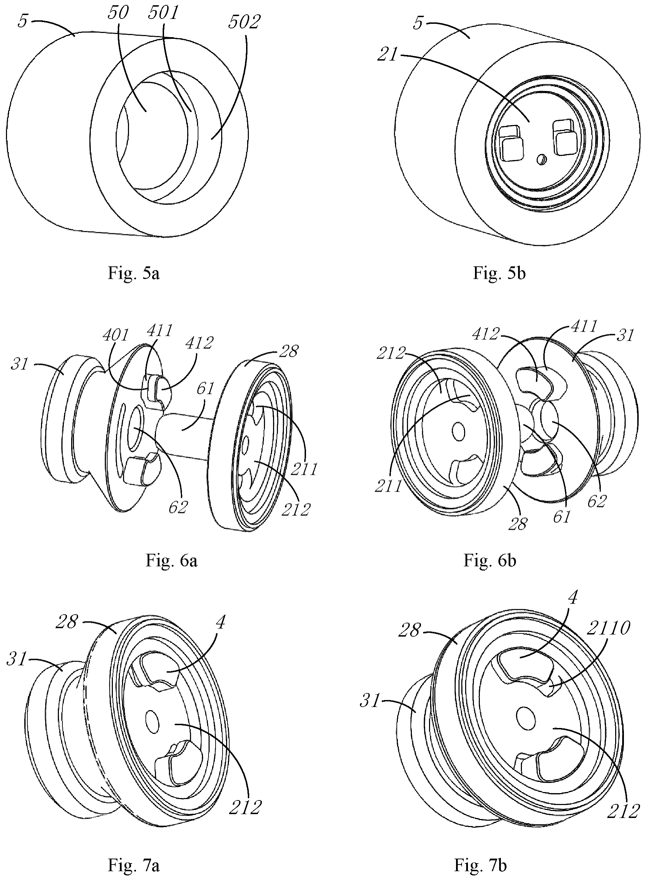

Referring to Figs. 6a to 7b, in another example, in the context of the proximal connector 31 being regarded as the first base body and the safety metal wire connector 28 as the second base body, the proximal connector 31 and the safety metal wire connector 28 are configured to be attached to each other along the axial direction of the pacemaker body 2. In this example, the connecting member 4 similarly includes an engagement post 411 and an engagement body 412. The engagement post 411 extends along the axial direction of the pacemaker body 2. An end of the engagement post 411 away from the pacemaker body 2 is fixedly attached to the proximal connector 31, and the other end thereof facing toward the pacemaker body 2 is fixedly attached to the engagement body 412. The engagement body 412 extends circumferentially with respect to the pacemaker body 2, and a first stop surface 401 is provided at a surface of the engagement body 412 facing away from the pacemaker body 2. The safety metal wire connector 28 has a first through hole 211, and an end face of the safety metal wire connector 28 away from the proximal connector 31 is a second stop surface 212. The engagement post 411 and the engagement body 412 are configured to rotate circumferentially with respect to the pacemaker body 2 with the proximal connector 31, thereby causing rotation of the first stop surface 401.

It should be noted that, in the example of Figs. 6a to 7b, the proximal connector 31 is configured to rotate circumferentially with respect to the pacemaker body 2, rather than displacing perpendicularly to the axial direction of the pacemaker body 2, relative to safety metal wire connector 28. When proximal connector 31 rotates circumferentially about the axis of the pacemaker body 2, the engagement post 411 and the engagement body 412 will rotate with the proximal connector 31. Conformably, the engagement post 411 and the engagement body 412 together have an L-shaped circumferential cross-section, which allows the engagement post 411 and the engagement body 412 to engage within the first through hole 211 as a result of rotation, thereby allowing the first stop surface 401 of the engagement body 412 to abut against the second stop surface 212.

Optionally, the first and second base bodies may mate with each other along the axis of the pacemaker body 2 in the form of protrusion and recess. When this happens, the first base body is restricted from rotating circumferentially with respect to the pacemaker body 2 and/or displacing perpendicularly to the axial direction of the pacemaker body 2 relative to the second base body. The protrusion and recess matching between the first base body and the second base body is achieved simultaneously as they are connected by the connecting member 4, which enables efficient assembly and enhanced shear resistance at the joint of the first and second base bodies. Specifically, one of the first and second base bodies may comprise a locating protrusion 61, and the other may comprise a locating hole 62. The locating protrusion 61 can be inserted into the locating hole 62 to restrict the first base body from moving perpendicularly to the axial direction of the pacemaker body 2 relative to the second base body. In the example of Figs. 6a to 7b, the safety metal wire connector 28 comprises the locating protrusion 61, and the proximal connector 31 comprises the locating hole 62. The locating protrusion 61 projects and extends along an axis of the safety metal wire connector 28 toward the proximal connector 31 and has a circular cross-section. The locating hole 62 is depressed along an axis of the proximal connector 31 and also has a circular cross-section. When the locating protrusion 61 is inserted into the locating hole 62, their side walls will abut against each other. This restricts movement of proximal connector 31 relative to safety metal wire connector 28 in a direction perpendicular to the axial direction of pacemaker body 2, while allowing circumferential rotation between them. This facilitates engagement of proximal connector 31 with safety metal wire connector component 28 via rotation.

On the basis of the above description of the leadless pacemaker, in the first embodiment of the present invention, there is also a method for assembling the leadless pacemaker, which includes the steps of:
S1: bringing the first base body into abutment with an end of the second base body away from the pacemaker body 2 along the axial direction of the pacemaker body 2;
S2: inserting a second end of the connecting member 4 through the second base body and bringing it into abutment with an end face of the second base body away from the first base body along the axial direction of the pacemaker body 2, thereby restricting the first base body from displacing relative to the second base body along the axial direction of the pacemaker body 2.

Optionally, inserting the second end of the connecting member 4 through the second base body and bringing it into abutment with the end face of the second base body away from the first base body along the axial direction of the pacemaker body 2 may specifically include:
in step S21, displacing the first base body perpendicularly to the axial direction of the pacemaker body 2 or circumferentially rotating it with respect to the pacemaker body so that the first stop surface 401 of the engagement body 412 of the connecting member 4 comes into abutment with the second stop surface 212 of the second base body, thereby restricting the first base body from displacing relative to the second base body along the axial direction of the pacemaker body 2. The first base body may be displaced perpendicularly to the axial direction of the pacemaker body 2, as described in the foregoing specific implementations in connection with Figs. 2 to 4, and the first base body may be rotated circumferentially with respect to the pacemaker body, as described in the above specific implementations in connection with Figs. 6a to 7b.

The method may further include attachment enhancement. It should be noted that the attachment enhancement may be accomplished by at least one of various approaches as exemplified below.

The attachment enhancement may be accomplished by applying a medical adhesive between the first and second base bodies along the axial direction of the pacemaker body 2. In one example, prior to assembly and attachment, an epoxy resin adhesive for medical use, such as Loctite^{®} M-31CL (a two-component AB epoxy resin adhesive for medical use), may be applied to opposite end faces of the electrode base 15 and the distal shell connector 21 (i.e., the aforementioned first end face 150 and second end face 210), as shown in Fig. 4a. After that, the L-shaped engagement post 411 and the engagement body 412 may be inserted through the first through hole 211, as shown in Fig. 4b. The electrode base 15 may be then displaced perpendicularly to the axial direction of the pacemaker body 2 (e.g., in the direction indicated by the arrow of Fig. 4b), bringing the first stop surface 401 into abutment with the second stop surface 212, as shown in Fig. 4c.

Alternatively, in the case of the first through hole 211 being provided in the second base body, the attachment enhancement may be accomplished by applying a medical adhesive between the connecting member 4 and the first through hole 211, or by welding them to each other. The medical adhesive may be an epoxy resin adhesive for medical use, such as Loctite^{®} M-31CL (a two-component AB epoxy resin adhesive for medical use). Optionally, as shown in Figs. 2 and 3, a side wall of the engagement post 411 and an inner circumferential wall of the first through hole 211, which are intended to interact with each other, may be additionally fixed by bonding with a medical adhesive, or by welding. Further, the first stop surface 401 and the second stop surface 212 may also be additionally fixed by bonding with a medical adhesive, or by welding.

As an example, before the engagement post 411 and the engagement body 412 are inserted through the first through hole 211, an epoxy resin adhesive for medical use may be applied to an outer circumferential wall of the engagement post 411, or to the inner circumferential wall of the first through holes 211. In some other examples, an epoxy resin adhesive for medical use may be applied to the first stop surface 401, or to the second stop surface 212. The epoxy resin adhesive may cure after the engagement post 411 is inserted through the first through hole 211, resulting in significant improvements in attachment reliability and strength. In some other examples, after the engagement post 411 and the engagement body 412 are inserted through the first through hole 211 and then moved in place with the electrode base 15, an epoxy resin adhesive for medical use may be applied around the engagement post 411. Part of the epoxy resin adhesive may spread between the engagement post 411 and the first through hole 211 and between the first stop surface 401 and the second stop surface 212.

Referring to Figs. 5a and 5b, in the present embodiment, there is also provided an assembly aid 5, which allows for rapid assembly of the electrode base 15 and the distal shell connector 21. The assembly aid 5 comprises an axial cavity 50, the axial cavity 50 opens at both ends and, inside the cavity 50, a circumferential stop step 501 and a circumferential inner stop wall 502 are provided. The stop step 501 is configured to abut against the electrode base 15 on the side thereof away from the distal shell connector 21. The inner stop wall 502 is configured to restrict radial position of the electrode base 15 and the distal shell connector 21. In an example, each of the electrode base 15 and the distal shell connector 21 has a circular outer circumference, which matches in shape to the inner stop wall 502 and has an outer diameter equal to or slightly less than an inner diameter of the inner stop wall 502.

After the engagement post 411 and the engagement body 412 are inserted through the first through hole 211 and then moved in place with the electrode base 15 (as shown in Fig. 4c), the electrode base 15 and the distal shell connector 21 may be together placed within the cavity 50 of the assembly aid 5, and an epoxy resin adhesive for medical use may be then applied around the engagement post 411. In this process, confined by the inner stop wall 502, the electrode base 15 and the distal shell connector 21 can be retained at correct positions, and until the epoxy resin adhesive cures, attachment of the electrode base 15 and the distal shell connector 21 is completed.

### Embodiment 2

With continued reference to Figs. 1 to 7b, in a second embodiment of the present invention, there are provided a leadless pacemaker and a method of assembly thereof, which are essentially similar to the leadless pacemaker and method of the first embodiment, except for some differences. In the following, only these differences will be described, and the similarities between the two embodiments are not repeated.

As shown in Figs. 1 to 7b, the leadless pacemaker of the second embodiment differs from the leadless pacemaker of the first embodiment in that the connecting member 4 further includes a securing member (not shown), which fills a gap between the first through hole 211 and the engagement post 411 to prevent the engagement post 411 from moving in the first through hole 211 perpendicularly to the axial direction of the pacemaker body 2 or circumferentially with respect to the pacemaker body 2. In the arrangement of the securing member, the engagement perpendicular to the axial direction of the pacemaker body 2 and the circumferential engagement can prevent the engagement post 411 from moving backwards once it is engaged. As shown in Fig. 4c, after the L-shaped engagement post 411 and the engagement body 412 is inserted through the first through hole 211 and moved together with the electrode base 15 perpendicularly to the axial direction of the pacemaker body 2, a residual space 2110 will be left in the first through hole 211. The securing member may fill the residual space 2110 to prevent the engagement post 411 from moving backwards perpendicularly to the axial direction of the pacemaker body 2, thus reliably securing the engagement post 411 in the first through hole 211. It should be noted that the securing member may be provided as a component separately molded, such a plug part, or as a structure resulting from the curing of an amorphous material, such as an epoxy resin adhesive, without departing from the scope of the present invention.

Correspondingly, after the first stop surface 401 of the engagement body 412 in the connecting member 4 is brought into abutment with the second stop surface 212 of the second base body as a result of moving the first base body perpendicularly to the axis of the pacemaker body 2 or circumferentially rotating it with respect to the pacemaker body in step S21, the method of this embodiment further includes:
in step S22, filling a gap between the first through hole 211 in the second base body and the engagement post 411 with the securing member, thereby preventing the engagement post 411 from moving in the first through hole 211 perpendicularly to the axis of the pacemaker body 2 or circumferentially with respect to the pacemaker body.

The gap in step S22 is the aforementioned residual space 2110. After the residual space is filled or packed with the securing member, the securing member may be fastened in the residual space 2110 by welding or adhesive bonding. Additionally, in order to achieve a good seal, an epoxy resin adhesive for medical use may be applied to the outer circumferential wall of the engagement post 411.

In alternative examples, the residual space 2110 may be filled with an epoxy resin adhesive for medical use, which cures at a later time to form the securing member.

### Embodiment 3

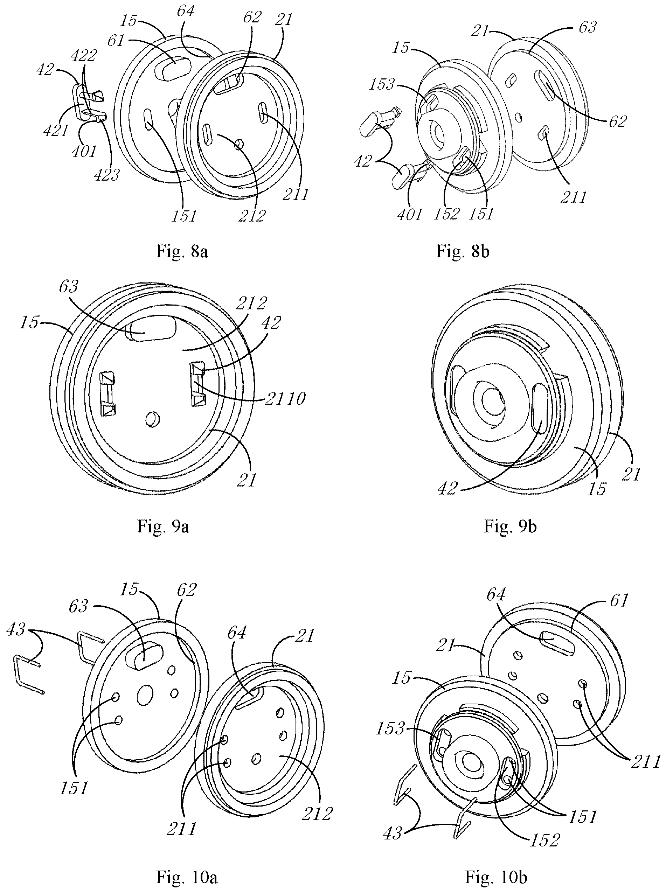

Referring to Figs. 8a to 9b, in a third embodiment of the present invention, there are provided a leadless pacemaker and a method of assembly thereof, which are essentially similar to the leadless pacemaker and method of the first embodiment, except for some differences. In the following, only these differences will be described, and the similarities between the two embodiments are not repeated.

As shown in Figs. 8a to 9b, in the leadless pacemaker of the third embodiment, the first and second base bodies and the connecting member 4 are structured and attached differently from those of the first embodiment. Specifically, in the third embodiment, instead of being attached to the first base body, the connecting member 4 is inserted through, and brought into abutment with, both the first and second base bodies, thereby restricting axial displacement of the first base body relative to the second base body. Referring to Figs. 8a to 9b, in the context of the electrode base 15 being regarded as the first base body and the distal shell connector 21 as the second base body, the electrode base 15 has a second through hole151 allowing the insertion of the connecting member 4 therethrough and a third stop surface 152 facing away from the distal shell connector 21. The second through hole 151 extends through the electrode base 15 parallel to the axis of the pacemaker body 2, and the connecting member 4 is configured to abut against both the third stop surface 152 and the second stop surface 212, restricting the electrode base 15 from moving relative to the distal shell connector 21 along the axis of the pacemaker body 2. In this case, the connecting member 4 can be detached, facilitating assembly.

When the direction of the second through hole 151 is parallel to the axis of the pacemaker body 2, each connecting member 4 corresponds to at least one second through hole 151. The two ends of the connecting member 4 sequentially pass through the corresponding second through hole 151 and the first through hole 211 at their respective positions, then it abuts against the second stop surface 212. The portion of the connecting member 4 on the side of the electrode base 15 away from the distal shell connector 21 abuts against the third stop surface 152.

As shown in Figs. 8a to 9b, in an example, each connecting member 4 corresponds to one second through hole 151 and includes an elastic engagement element 42, and the first stop surface 401 is biased by elastic potential energy perpendicularly to the axis of the pacemaker body 2 into abutment with the second stop surface 212. Optionally, the elastic engagement element 42 may be substantially a π-shaped element including a base 421 and two elastic legs 422. The base 421 has an end face facing toward the third stop surface 152, which is intended to be brought into abutment against the third stop surface 152. Each of the legs 422 is attached, at one end away from the pacemaker body 2, to said end face of the base 421 and free at the other end. Each leg 422 is provided with a reverse-lock boss away from the other leg. The first stop surface 401 is provided at a surface of the reverse-lock boss 423 facing away from the pacemaker body 2. Compared with the bendable wire described in detail below (see Figs. 10a to 11b), the elastic engagement element 42 dispenses with bending operation(s) during assembly, resulting in improved assembly efficiency.

Correspondingly, the first through hole 211 axially extends through the distal shell connector 21, and the second through hole151 axially extends through the electrode base 15. The first through hole 211 and the second through hole 151 are both oblong. After the electrode base 15 and the distal shell connector 21 are brought into abutment with each other along the aforesaid axis, the legs 422 may be inserted through both the second through hole151 and the first through hole 211. During passage through the second through hole 151 and the first through hole 211, the reverse-lock bosses 423 are compressed by the first and second through holes, causing them to move toward each other. After the reverse-lock bosses 423 emerge from the first through hole 211, it will not be confined by the compressive constraint. The two legs 422 then release their elastic energy. As a result, they will deflect away from each other both into engagement with the second stop surface 212, bringing the first stop surfaces 401 into abutment with the second stop surface 212, as shown in Figs. 9a and 9b. At this point, with additional reference to Figs. 8b and 9b, the end face of the base 421 will come into abutment with the third stop surface 152. In this way, the elastic engagement element 42 can lock the electrode base 15 and the distal shell connector 21 against each other, accomplishing their assembly.

Further, an epoxy resin adhesive for medical use may be applied to locations, where the elastic engagement element 42 comes into contact with the second through hole 151 and the first through hole 211. Furthermore, a securing member may be disposed in a residual space 2110 left in the first through hole 211 upon the engagement of the elastic engagement element 42 (see Fig. 9a). The securing member may be disposed and secured in the residual space 2110 in a similar manner as in the preceding embodiment, and description thereof is not repeated here.

It will be understood that the elastic engagement element 42 with two legs 422 is merely an exemplary implementation of the elastic engagement element, and the elastic engagement element is not limited to being so implemented. In some other implementations, the elastic engagement element 42 may include multiple legs 422 distributed in a certain pattern, such as circumferential. The multiple reverse-lock bosses 423 may be radially deflected outwards. Correspondingly, the second through hole 151 and the first through hole 211 may have a circular, quadrilateral or other shape, without limiting the scope of the present invention.

In the example of Figs. 8a to 9b, the leadless pacemaker includes two symmetrical elastic engagement elements 42. Correspondingly, the distal shell connector 21 has two first through holes 211, and the electrode base 15 has two second through holes 151. With this arrangement, statisfactory attachment strength can be provided. The elastic engagement elements 42 correspond to the respective first through holes 211 and to the respective second through holes 151.

It will be understood that any numbers of elastic engagement elements 42, second through holes 151 and first through holes 211 may be included, as desired in practical applications. For example, there may be more than two elastic engagement elements 42, which may be evenly distributed circumferentially around the center of the electrode base 15. More elastic engagement elements 42 mean higher attachment strength. The present invention is not particularly limited in this regard.

Further, referring to Fig. 8b, instead of providing the end face of the electrode base 15 away from the distal shell connector 21 directly as the third stop surface 152, as described earlier in this embodiment, the electrode base 15 may be provided with a recess 153 depressed toward an interior of the electrode base 15 , and the second through hole 151 may be formed in the third stop surface 152, and part of the connecting member 4 may be received in the recess 153. In particular, upon engagement of the elastic engagement element 42 with the first through hole 211, the base 421 may be received in the recess 153.

Preferably, an inner contour of the recess 153 may match to an outer contour of the base 421 in shape. For example, the inner contour shape of the recess 153 may be the same as, or slightly larger than, the outer contour shape of the base 421. Further, a depth of the recess 153 measured along the axis of the pacemaker body 2 may be not less than a thickness of the base 421 measured in the same direction. This can prevent the base 421 from being raised outside the recess 153 when the elastic engagement element 42 engages with the first through hole 211. With this arrangement, the use of the detachable connecting member 4 enables an overlap of the first and second base bodies to be utilized, allowing for full utilization of the limited space without modifying the size of the leadless pacemaker and thus allowing the leadless pacemaker to be made compact.

In some optional implementations, instead of being detachable from the electrode base 15 and inserted through both the second and first through holes 151, 211, one end of the elastic engagement element 42 may be fixedly attached to the electrode base 15. For example, the base 421 may be fixedly attached to the electrode base 15, while the legs 422 may have free ends, which may be passed through the first through hole 211 to allow engagement of the reverse-lock bosses 423 with the second stop surface 212, and the first stop surface 401 comes into abutment with the second stop surface 212.

As noted above, in this third embodiment, assembly is achieved by spreading of the engagement end of the connecting member 4 following its successive insertion through the second through hole151 and the first through hole 211. Therefore, relative displacement of the electrode base 15 and the distal shell connector 21 is not necessary, and accordingly, the electrode base 15 and distal shell connector 21 may mate each other along the axis of the pacemaker body 2 optionally in the form of a male/female pair, which can restrict the electrode base 15 from displacing perpendicularly to the axial direction of the pacemaker body 2 and/or from rotating along a circumferential direction of the pacemaker body 2 relative to the distal shell connector 21.

Referring to Figs. 8a to 9b, the electrode base 15 may have a locating protrusion 61, and the distal shell connector 21 may have a corresponding first locating hole 62. The locating protrusion 61 may be in one-to-one correspondence with the first locating hole 62. The locating protrusion 61 may project and extend toward the distal shell connector 21 parallel to an axis of the electrode base 15 and have an obround cross-section, while the first locating hole 62 may axially extend through the electrode base 15 and also have an obround cross-section. The locating protrusion 61 may be inserted into the first locating hole 62 so that their side walls abut against each other, preventing the electrode base 15 from displacing perpendicularly to the axis of the pacemaker body 2 or from rotating along a circumferential direction of the pacemaker body 2 relative to the distal shell connector 21. Meanwhile, this enables higher shear resistance of the joint between the electrode base 15 and the distal shell connector 21. Of course, in other implementations, the first locating hole 62 may also be replaced with a recess matching the locating protrusion 61 in shape while still providing the same benefits. Further description thereof is omitted herein, for the sake of brevity.

Further, one of the electrode base 15 and the distal shell connector 21 comprises a circumferential locating boss 63, and the other comprises a locating depression 64. The locating boss 63 can be snuggly inserted into the locating depression 64. Referring to Figs. 8a and 8b, in one implementation, the locating boss 63 is provided on the distal shell connector 21, and protrudes toward the electrode base 15, and the locating depression 64 is correspondingly provided in the electrode base 15. An inner contour of the locating depression 64 matches with an outer contour of the locating boss 63 in shape. For example, both may be circular. The locating boss 63 can be inserted into the locating depression 64, and abuts against the inner contour of the locating depression 64. With this arrangement, after the locating boss 63 is inserted into the locating depression 64, the electrode base 15 will be restricted from displacing perpendicularly to the axis of the pacemaker body 2 relative to the distal shell connector 21 while still being allowed to rotate circumferentially with respect to the pacemaker body 2. During assembly of the electrode base 15 and the distal shell connector 21, the locating boss 63 can work with the locating depression 64 to allow them to achieve both radial and axial locating with respect to the pacemaker body 2, facilitating subsequent attachment of the connecting member 4.

Alternatively, the outer contour of the locating boss 63 and the inner contour of the locating depression 64 may be non-circular, such as polygonal or elliptic. In this case, the locating boss 63 and the locating depression 64 can work together to further restrict the above two from rotating circumferentially with respect to the pacemaker body 2 relative to each other.

It will be understood that either or both of the locating protrusion 61/locating hole 62 and locating boss 63/locating depression 64 combinations may be provided, and those skilled in the art can make an appropriate choice depending on how the connecting member 4 achieves engagement. In particular, when both combinations are provided, there is a clearance left between the locating boss 63 and the locating depression 64, in order to prevent the two from forming an interference fit.

Correspondingly, in the case of the connecting member 4 being detachable, in step S2 of the method of this embodiment, the second end of the connecting member 4 is inserted through the second base body along the axis of the pacemaker body 2 and brought into abutment with the end face of the second base body away from the first base body. Specifically, this may include:
in step S23, inserting the connecting member 4 successively through the second through hole 151 in the first base body and the first through hole 211 in the second base body and bringing it into abutment with both the third stop surface 152 of the first base body and the second stop surface 212.

Optionally, in the case of the connecting member 4 including the elastic engagement element 42, step S23 may specifically include: elastic opposite deflection of the two legs 422 of the elastic engagement element 42 as a result of inserting them successively through the second through hole151 and the first through hole 211, which brings the reverse-lock bosses 423 of the legs 422 into engagement with the second base body, thereby restricting displacement of the first base body along the axis of the pacemaker body 2 relative to the second base body.

The method may further include attachment enhancement. In the case of the connecting member 4 being detachable, when the second through hole151 is provided in the first base body, the attachment enhancement may be accomplished by applying a medical adhesive between the connecting member 4 and the second through hole151. The medical adhesive may be an epoxy resin adhesive for medical use, such as Loctite^{®} M-31CL (a two-component AB epoxy resin adhesive for medical use).

In the case of the connecting member 4 being detachable, the attachment enhancement may also be accomplished by applying a medical adhesive between the first and second base bodies along the axis of the pacemaker body 2. Still alternatively, the attachment enhancement may be accomplished by applying a medical adhesive between the connecting member 4 and the first through hole 211, or by welding them together.

In the case of the connecting member 4 including the elastic engagement element 42, the elastic engagement element 42 may be optionally made of a metallic material, such as 316L stainless steel, TA2 titanium or NiTi-01 nickel-titanium alloy. Preferably, instead of adhesive bonding, after the elastic engagement element 42 is inserted through the second through hole151 and the first through hole 211 and engages around the first through hole 211, it may be attached to the second through hole151 and the first through hole 211 by welding (e.g., laser welding). This can result in even higher attachment strength and tensile resistance.

### Embodiment 4

Referring to Figs. 10a to 11b, in a fourth embodiment of the present invention, there are provided a leadless pacemaker and a method of assembly thereof, which are essentially similar to the leadless pacemaker and method of the third embodiment, except for some differences. In the following, only these differences will be described, and the similarities between the two embodiments are not repeated.

As shown in Figs. 10a to 11b, in the leadless pacemaker of the fourth embodiment, the connecting member 4 is structured and attached differently from the connecting member 4 of the third embodiment. Specifically, as shown in Figs. 10a to 11b, in the fourth embodiment, the connecting member 4 includes a bendable wire 43, which is inserted through the first through hole 211 and then bent into abutment with the second stop surface 212 of the second base body, thereby restricting the first base body from displacement along the axis of the pacemaker body 2 relative to the second base body. That is, the first stop surface 401 is formed by bending the bendable wire 43 after it passes through the first through hole 211. In some implementations, the bendable wire 43 is a metal wire made of, for example, 316L stainless steel, PtLr20 platinum-iridium alloy, NiTi-01 nickel-titanium alloy or the like. Preferably, the bendable wire 43 may have, for example, a circular, rectangular, elliptical or other cross-section. In some implementations, the bendable wire 43 is a flexible wire, such as a multi-strand stainless steel wire, or a multi-strand nickel-titanium alloy wire. In Figs. 10a to 11b, the electrode base 15 is shown as the first base body and the distal shell connector 21 as the second base body. The electrode base 15 has a second through hole 151 and a third stop surface 152 facing away from the distal shell connector 21. The direction of the second through hole 151 is parallel to the axis of the pacemaker body 2, and the distal shell connector 21 has a first through hole 211 and a second stop surface 212 facing away from the electrode base 15.

After the electrode base 15 is brought into abutment with an end of the distal shell connector 21 away from the pacemaker body 2 along the axis of the pacemaker body 2, the bendable wire 43 may be inserted successively through the second through hole 151 and the first through hole 211, and a portion thereof that is inserted beyond the first through hole 211 may be then bent into abutment with the second stop surface 212. That is, this portion provides the first stop surface 401. The bendable wire 43 may have another portion, which is not inserted through the second through hole 151 and brought into abutment with the third stop surface 152. In this way, the electrode base 15 and the distal shell connector 21 can be assembled and locked via the bendable wire 43.

Optionally, each bendable wires 43 is associated with two second through holes 151. In this case, opposite ends of each bendable wire 43 may be inserted successively through the associated respective second through holes 151 and associated respective first through holes 211 and then bent to form first stop surface 401 which abuts against the second stop surface 212, concurrently with a portion of the bendable wire 43, which remains on the side of the electrode base 15 away from the distal shell connector 21, coming into abutment with the third stop surface 152. In this way, each bendable wire 43 has a middle portion in abutment with the electrode base 15 and end portion in abutment with the distal shell connector 21. This allows for both firmer attachment and ease of assembly. Specifically, axially opposite ends of each bendable wire 43 may be both inserted through respective first through holes 211 and then bent. It will be understood that this requires the presence of both a pair of second throughs 151 in the electrode base 15 and a pair of first through holes 211 in the distal shell connector 21 for each bendable wire 43.

Of course, in alternative implementations, only one axial end of each bendable wire 43 may be inserted through a first through hole 211 and bent. In this case, for each bendable wire 43, the presence of only one second through hole 151 in the electrode base 15 and only one first through hole 211 in the distal shell connector 21 is required. For example, each bendable wire 43 may have one end, which is enlarged and could not be passed through a second through hole 151 or first through hole 211, while the other end thereof can be inserted through the second through hole 151 and the first through hole 211 and then bent. This also enables assembly and locking of the electrode base 15 and the distal shell connector 21.

Optionally, the electrode base 15 may comprises a recess 153 (see Fig. 10b), and the third stop surface 152 may be provided by a bottom surface of the recess 153. In this case, after a portion of the bendable wire 43 is inserted beyond the first through hole 211, another portion thereof, which remains not inserted though the second through hole 151, may be received in the recess 153. Preferably, an inner contour of the recess 153 may be slightly larger than an outer contour of the other portion of the bendable wire 43 that remains not inserted though the second through hole 151. Preferably, the recess 153 may have a depth not less than an outer diameter of the bendable wire 43. It will be understood that that the depth of the recess 153 refers to a dimension thereof measured along the axis of the pacemaker body 2. In this way, after the portion of the bendable wire 43 inserted beyond the first through hole 211 is bent, the other portion that remains not inserted through the second through hole 151 will not protrude out of the recess 153. With this arrangement, an overlap of the first and second base bodies can be utilized to allow for full utilization of the limited space without modifying the size of the leadless pacemaker.

In some optional implementations, instead of being detachable from the electrode base 15 and inserted through both the second and first through holes 151, 211, one end of the bendable wire 43 may be fixedly attached to the electrode base 15, while the other end may be a free end, which can be inserted through the first through hole 211. Additionally, a portion at the free end inserted beyond the first through hole 211 may be bent into abutment with the second stop surface 212.

Optionally in this embodiment, in addition to assembly and locking of the electrode base 15 and the distal shell connector 21 achieved by the bendable wire 43, a locating protrusion 61 and/or a locating boss 63 may be provided on one of the electrode base 15 and the distal shell connector 21, and a locating hole 62 and/or a locating depression 64 may be provided in the other, in order to restrict the electrode base 15 from displacing perpendicularly to the axis of the pacemaker body 2 and/or from rotating along circumferential direction of the pacemaker body 2 relative to the distal shell connector 21. For more details in this regard, reference can be made to the above description in connection with the third embodiment, and further description thereof is omitted here.

In one example, as shown in Figs. 10a to 11b, the leadless pacemaker includes two bendable wires 43 and, accordingly, four first through holes 211 in the distal shell connector 21 and four second through holes 151 in the electrode base 15. With this arrangement, desired attachment strength can be obtained. It will be understood that any numbers of bendable wires 43, second through holes 151 and first through holes 211 may be included, as desired in practical applications, without departing from the scope of the present invention.

Correspondingly, when the connecting member 4 comprises bendable wires 43, compared with the method of the third embodiment, step S23 in the method of this embodiment may include:
inserting both ends of the bendable wire 43 successively through the respective second through holes 151 and the respective first through holes 211 and bending them into abutment with the second stop surface 212 of the second base body, thereby restricting the first base body from displacement along the axis of the pacemaker body 2 relative to the second base body.

In this embodiment, the method may further include attachment enhancement. Regarding the connecting member 4 comprises bendable wires 43, in case of the second through hole 151 being provided in the first base body, the attachment enhancement may be accomplished by applying a medical adhesive between the connecting member 4 and the second through hole 151.

In an optional implementation, a medical adhesive, such as an epoxy resin adhesive for medical use, may be applied to at least one of an outer circumferential wall of the bendable wire 43, an inner circumferential wall of the second through hole 151, an inner circumferential wall of the first through hole 211, the first stop surface 401, the second stop surface 212 and the third stop surface 152, to adhesively bond the bendable wire 43 to the electrode base 15 or to the distal shell connector 21.

In an alternative implementation, the portion of the bendable wire 43 that is inserted beyond the first through hole 211 and bent is attached to the distal shell connector 21 by welding. That is, in addition to being brought into abutment with the second stop surface 212, the first stop surface 401 is additionally fixed to the second stop surface 212 by welding. In this way, even higher attachment strength of the electrode base 15 and the distal shell connector 21 can be achieved.

### Embodiment 5

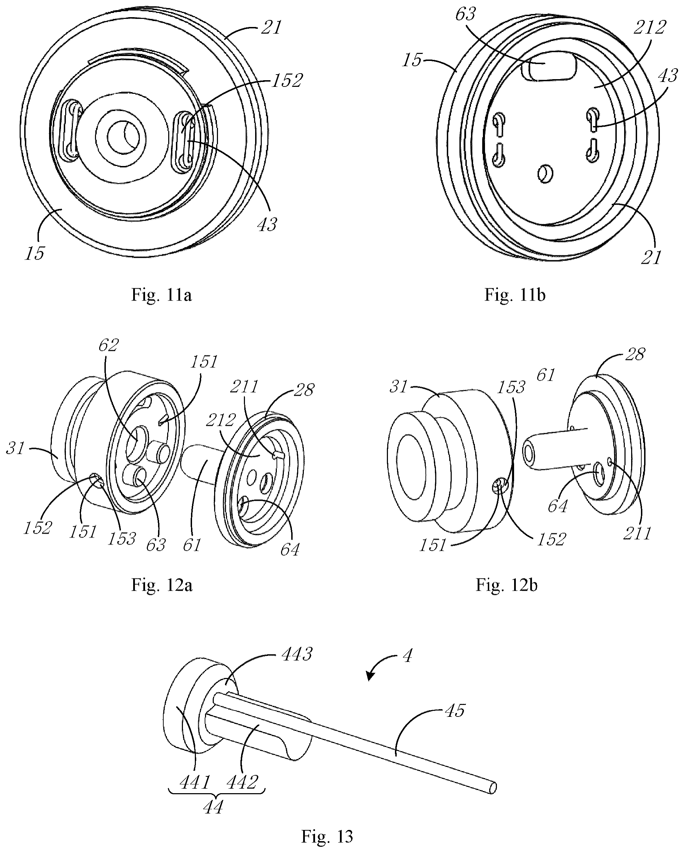

Referring to Figs. 12a to 14b, in a fifth embodiment of the present invention, there are provided a leadless pacemaker and a method of assembly thereof, which are essentially similar to the leadless pacemaker and method of the fourth embodiment, except for some differences. In the following, only these differences will be described, and the similarities between the two embodiments are not repeated.

As shown in Figs. 12a to 14b, in the leadless pacemaker of the fifth embodiment, direction of the second through hole 151 is different from that of the fourth embodiment, and the connecting member 4 is structured and attached differently from that of the fourth embodiment. These differences are described below with reference to Figs. 12a to 14d, in which the proximal connector 31 is defined as the first base body and the safety metal wire connector 28 is defined as the second base body.

The proximal connector 31 comprises a third stop surface 152 located on its circumference and the second through hole 151 configured for insertion of the connecting member therethrough, the second through hole extends perpendicularly to the axis of the pacemaker body 2. The safety metal wire connector 28 comprises a first through hole 211 extending therethrough along the axis of the pacemaker body 2 and a second stop surface 212 facing away from the proximal connector 31. The connecting member 4 is configured to abut against both the third stop surface 152 and the second stop surface 212 to restrict the proximal connector 31 from displacement along the axis of the pacemaker body 2 relative to the safety metal wire connector 28.

The second through hole 151 that extends perpendicularly to the axis of the pacemaker body 2 communicates with the first through hole 211.

As shown in Fig. 13, the connecting member 4 includes a plug part 44 and a bendable part 45, which are attached to each other. A free end of the bendable part 45 is successively inserted through the second through hole 151 and the first through hole 211 and then bent to form a first stop surface which abuts against the second stop surface 212, concurrently with the plug part 44 coming into abutment with the third stop surface 152. After being inserted through the second through hole151, the bendable part 45 is further inserted through the first through hole 211 and then again bent so as to come into abutment with the second stop surface 212. In this way, the proximal connector 31 and the safety metal wire connector 28 can be assembled and locked. As shown in Fig. 13, in one example, the plug part 44 may be a metal component made of, for example, 316L stainless steel, PtLr20 platinum-iridium alloy, NiTi-01 nickel-titanium alloy or the like. The bendable part 45 may be a metal wire made of, for example, 316L stainless steel, PtLr20 platinum-iridium alloy, NiTi-01 nickel-titanium alloy or the like. Preferably, the bendable part 45 may have a circular, rectangular, elliptical or other cross-section. Alternatively, the bendable part 45 may be a flexible wire, such as a multi-strand stainless steel wire, or a multi-strand nickel-titanium alloy wire. Optionally, the plug part 44 may include a main body 441 and a coupling part 442. The main body 441 is substantially in the shape cylindrical or frusto-conical in shape and has a plug end face 443, and the coupling part 442 is fixedly attached to the plug end face 443. The coupling part 442 may have a curved shape matching the cross-section of the bendable part 45. Preferably, the bendable part 45 is attached to the plug part end face 443 by welding (e.g., laser welding).

Optionally, the proximal connector 31 may comprises a recess 153 depressed toward the proximal connector 31, and the third stop surface 152 may be provided by a bottom surface of the recess 153. The second through hole 151 may be formed in the third stop surface 152, and part of the connecting member 4 may be received in the recess 153. The recess 153 may have an inner contour shape matching an outer contour shape of the plug part 44. The plug part 44 may be inserted into the recess 153 so that the end face 443 abuts against the third stop surface 152. The main body 441 can be completely received in the recess 153 without expanding a radial size of the leadless pacemaker, allowing the leadless pacemaker to be made compact.

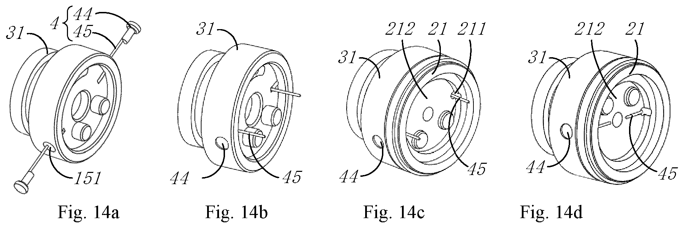

The bendable part 45 is first inserted into the second through hole 151 through an opening thereof (see Fig. 14a). After being passed through the second through hole 151, the bendable part 45 can be bent to change the extending direction, then exiting the second through hole 151(see Fig. 14b). In order to facilitate bending of the bendable part 45, the bendable part 45 may be obliquely inserted into the second through hole151, and then may be aligned with the first through hole 211 of the safety metal wire connector 28, thereby inserting the bendable part 45 through the first through hole 211, enabling the safety metal wire connector 28 to come into abutment with the proximal connector 31 (see Fig. 14c). Finally, the bendable part 45 is again bent so as to abut against the second stop surface 212 (see Fig. 14d). It will be understood that the portion of the bendable part 45 bent into abutment with the second stop surface 212 provides the first stop surface 401.

Since the second through hole151 extends perpendicularly to the axis of the pacemaker body 2, step S23 in the method of this embodiment differs from that of the fourth embodiment. Specially, step S23 in this embodiment includes:
inserting the bendable part 45 of the connecting member 4 through the second through hole151 perpendicularly to the axis of the pacemaker body 2 and then bending it so that the bendable part 45 extends along the axis of the pacemaker body 2; and inserting the bendable part 45 through the first through hole 211 and then bending it to abut with the second stop surface 212, thereby restricting the first base body from displacement along the axis of the pacemaker body 2 relative to the second base body.

In this embodiment, the method may further include attachment enhancement. In case of the second through hole151 that extends perpendicularly to the axis of the pacemaker body 2 being provided in the first base body, the attachment enhancement may be accomplished by applying a medical adhesive between the connecting member 4 and an inner wall of the second through hole 151. The medical adhesive may be an epoxy resin adhesive for medical use, such as Loctite^{®} M-31CL (a two-component AB epoxy resin adhesive for medical use).

In one example, the attachment enhancement may be accomplished by applying a medical adhesive, such as an epoxy resin adhesive for medical use, to at least one of the plug end face 443, the third stop surface 152, an inner circumferential wall of the recess 153 and an outer circumferential wall of the main body 441 to enhance attachment strength and provide a seal.

In an optional implementation, the portion of the bendable part 45 that is inserted beyond the first through hole 211 and is bent is attached to the safety metal wire connector 28 by welding. That is, in addition to being brought into abutment with the second stop surface 212, the first stop surface 401 is additionally attached to the second stop surface 212 by welding (i.e., the attachment enhancement). In this way, even higher attachment strength of the proximal connector 31 and the safety metal wire connector 28 can be achieved.

In an alternative implementation, the attachment enhancement may be accomplished by applying a medical adhesive, such as an epoxy resin adhesive for medical use, to at least one of an outer circumferential wall of the bendable part 45, an inner circumferential wall of the first through hole 211, the first stop surface 401 and the second stop surface 212, in order to additionally enhance attachment strength of the proximal connector 31 and the safety metal wire connector 28.

It should be noted that any scenario described above in the context that the electrode base 15 is regarded as the first base body and the distal shell connector 21 as the second base body is equally applicable to a scenario in which the proximal connector 31 is considered as the first base body and the safety metal wire connector 28 as the second base body. Similarly, any scenario described above in the context that the proximal connector 31 is regarded as the first base body and the safety metal wire connector 28 as the second base body is equally applicable to a scenario in which the electrode base 15 is considered as the first base body and the distal shell connector 21 as the second base body.

As described above, the present invention provides a leadless pacemaker and a method of assembly thereof. The leadless pacemaker includes a pacemaker body, a first base body, a second base body and a connecting member. The first base body is attached by the connecting member along an axis of the pacemaker body to the second base body on a side thereof away from the pacemaker body. A first end of the connecting member is attached to the first base body, and a second end of the connecting member is inserted through the second base body and brought into abutment with an end face of the second base body away from the first base body along the axis of the pacemaker body, thereby restricting the first base body from displacement along the axis of the pacemaker body relative to the second base body. With this arrangement, the use of the connecting member enables an overlap of the first and second base bodies to be utilized, allowing for full utilization of the limited space and effectively enhancing axial tensile strength of the leadless pacemaker and attachment strength of its components, thereby satisfying the strength requirements of implantation, retrieval and long-term implantation of the leadless pacemaker without modifying the size thereof while taking into account ease of assembly.

It should be noted that the foregoing embodiments may be combined in any suitable combination. The description presented above is merely that of some preferred embodiments of the present invention and is not intended to limit the scope thereof in any sense. Any and all changes and modifications made by those of ordinary skill in the art based on the above teachings fall within the scope of the invention.

## Claims

1. A leadless pacemaker, comprising a pacemaker body, a first base body, a second base body and a connecting member, wherein the first base body is attached to a side of the second base body away from the pacemaker body by the connecting member along an axial direction of the pacemaker body, wherein a first end of the connecting member is attached to the first base body, and wherein a second end of the connecting member is inserted through the second base body and is brought into abutment with an end face of the second base body away from the first base body along the axial direction of the pacemaker body, thereby restricting the first base body from displacement with respect to the second base body along the axial direction of the pacemaker body.

2. The leadless pacemaker according to claim 1, wherein the connecting member comprises a first stop surface away from the pacemaker body, wherein the second base body comprises a first through hole configured for insertion of the connecting member therethrough, wherein an end face of the second base body away from the first base body is a second stop surface, wherein the first stop surface is brought into abutment with the second stop surface, thereby restricting the first base body from displacement with respect to the second base body along the axial direction of the pacemaker body.

3. The leadless pacemaker according to claim 2, wherein the connecting member comprises an engagement post and an engagement body, wherein the engagement post extends along the axial direction of the pacemaker body, wherein an end of the engagement post away from the pacemaker body is fixedly attached to the first base body, wherein an end of the engagement post facing toward the pacemaker body is fixedly attached to the engagement body, wherein the engagement body and the first base body are located on opposite sides of the second base body, wherein the engagement body extends perpendicularly to the axial direction of the pacemaker body or extends along a circumferential direction of the pacemaker body, wherein the first stop surface is provided at a surface of the engagement body away from the pacemaker body.

4. The leadless pacemaker according to claim 3, wherein when the engagement body extends perpendicularly to the axial direction of the pacemaker body, the first through hole abuts against a side wall of the engagement post, thereby restricting the engagement post from rotation in the first through hole.

5. The leadless pacemaker according to claim 4, wherein a shape of the first through hole is a polygon or an ellipse, wherein at least a portion of a circumferential side wall of the engagement post matches the shape of the first through hole.

6. The leadless pacemaker according to claim 3, wherein the connecting member further comprises a securing member, wherein the securing member fills a gap between the first through hole and the engagement post, thereby preventing the engagement post from moving perpendicularly to the axial direction of the pacemaker body or moving along a circumferential direction of the pacemaker body within the first through hole.

7. The leadless pacemaker according to claim 2, wherein the first base body comprises a third stop surface and a second through hole configured for insertion of the connecting member therethrough, wherein a direction of the second through hole is parallel or perpendicular to the axial direction of the pacemaker body, wherein the connecting member is brought into abutment with the third and second stop surfaces, thereby restricting the first base body from displacement with respect to the second base body along the axial direction of the pacemaker body.

8. The leadless pacemaker according to claim 7, wherein when a direction of the second through hole is parallel to the axial direction of the pacemaker body, the connecting member corresponds to at least one second through hole, wherein the connecting member are successively inserted through corresponding second through hole and the first through hole and is brought into abutment with the second stop surface, wherein a portion of the connecting member located on a side of the first base body and away from second base body is brought into abutment with the third stop surface.

9. The leadless pacemaker according to claim 8, wherein the connecting member is a bendable wire or an elastic engagement element.

10. The leadless pacemaker according to claim 9, wherein when the connecting member is an elastic engagement element, the elastic engagement element comprises a base and two elastic legs, wherein the base is brought into abutment with the third stop surface, wherein one end of each leg away from the pacemaker body is attached to base, and the other end of each leg is free end, wherein the free end of the leg comprises a reverse-lock boss extending away from the other leg, and wherein the first stop surface is provided at a surface of the reverse-lock boss away from the pacemaker body.

11. The leadless pacemaker according to claim 7, wherein when the direction of the second through hole is perpendicular to the axial direction of the pacemaker body, the second through hole communicates with the first through hole, wherein the connecting member comprises a plug part and a bendable part attached to the plug part, wherein a free end of the bendable part successively inserted through the second and first through holes, and is bended to form the first stop surface brought into abutment with the second stop surface , and wherein the plug part is brought into abutment with the third stop surface.

12. The leadless pacemaker according to claim 7, wherein the first base body comprises a recess depressed toward the first base body, wherein the third stop surface is formed at a bottom surface of the recess, wherein the second through hole is provided at the third stop surface, and wherein a portion of the connecting member is received in the recess.

13. The leadless pacemaker according to claim 7, wherein the first and second base bodies mate with each other along the axial direction of the pacemaker body in a form of protrusion and recess, thereby restricting the first base body from rotating with respect to the second base body along a circumferential direction of the pacemaker body and/or from displacing with respect to the second base body perpendicularly to the axial direction of the pacemaker body.

14. The leadless pacemaker according to claim 13, wherein one of the first and second base bodies comprises a locating protrusion, with the other comprising a locating hole, and wherein the locating protrusion is inserted into the locating hole.

15. The leadless pacemaker according to claim 14, wherein one of the first and second base bodies comprises a circumferential locating boss, with the other comprising a locating depression, wherein the locating boss is inserted into the locating depression.

16. The leadless pacemaker according to claim 1, comprising at least two connecting members, wherein the first base body is attached to the second base body through the at least two connecting members.

17. The leadless pacemaker according to claim 1, further comprising a pacemaker distal assembly, a pacemaker proximal assembly, wherein two first base bodies and two second base bodies are provided, wherein:
axially opposite ends of the pacemaker body are attached to corresponding second base bodies; and
the pacemaker distal assembly is attached to one of the first base bodies located on a distal end of the pacemaker body, and the pacemaker proximal assembly is attached to the other first base body located on a proximal end of the pacemaker body.

18. A method of assembly of the leadless pacemaker of any one of claims 1 to 17, comprising:
bringing the first base body into abutment with one end of the second base body away from the pacemaker body along the axial direction of the pacemaker body; and
inserting the second end of the connecting member through the second base body and bringing the second end of the connecting member into abutment with the end face of the second base body away from the first base body along the axial direction of the pacemaker body, thereby restricting the first base body from displacement with respect to the second base body along the axial direction of the pacemaker body.

19. The method according to claim 18, wherein inserting the second end of the connecting member through the second base body and bringing the second end of the connecting member into abutment with the end face of the second base body away from the first base body along the axial direction of the pacemaker body comprises:
moving the first base body perpendicularly to the axial direction of the pacemaker body or along a circumferential direction of the pacemaker body to bring the first stop surface of the engagement body in the connecting member into abutment with the second stop surface of the second base body, thereby restricting the first base body from displacement with respect to the second base body along the axial direction of the pacemaker body.

20. The method according to claim 19, wherein after moving the first base body perpendicularly to the axial direction of the pacemaker body or along a circumferential direction of the pacemaker body to bring the first stop surface of the engagement body in the connecting member into abutment with the second stop surface of the second base body, the method further comprises:
filling a securing member in a gap between a first through hole of the second base body and an engagement post, thereby preventing the engagement post from moving perpendicularly to the axial direction of the pacemaker body or along a circumferential direction of the pacemaker body within the first through hole.

21. The method according to claim 18, inserting the second end of the connecting member through the second base body and bringing the second end of the connecting member into abutment with the end face of the second base body away from the first base body along the axial direction of the pacemaker body comprises:
inserting the connecting member successively through the second through hole in the first base body and the first through hole in the second base body, thereby bringing the connecting member into abutment with each of the third stop surface of the first base body and the second stop surface.

22. The method according to claim 21, wherein the connecting member comprises a bendable wire, wherein opposite ends of the bendable wire are inserted successively through corresponding second and first through holes and then are bent into abutment with the second stop surface of the second base body, thereby restricting the first base body from displacement with respect to the second base body along the axial direction of the pacemaker body.

23. The method according to claim 21, wherein the connecting member comprises an elastic engagement element comprising two legs, wherein the two legs are inserted successively through corresponding second and first through holes and then elastically deflect away from each other to bring reverse-lock boss of the legs into engagement with the second base body, thereby restricting the first base body from displacement with respect to the second base body along the axial direction of the pacemaker body.

24. The method according to claim 18, further comprising performing an attachment enhancement by at least one of:
I: a medical adhesive applied between the first and second base bodies along the axial direction of the pacemaker body;
II: when the first through hole is provided in the second base body, a medical adhesive or welding is applied between the connecting member and the first through hole;
III: when the second through hole is provided in the first base body, a medical adhesive applied between the connecting member and the second through hole.
